(19) Europäisches Patentamt
European Patent Office
Office européen des brevets

(11) Publication number: **0 224 088 A2**

# (12) EUROPEAN PATENT APPLICATION

(21) Application number: 86115386.4

(22) Date of filing: 06.11.86

(51) Int. Cl.[4]: C07K 1/04 , C07K 5/02 , C07K 5/06

(30) Priority: 28.11.85 IT 2301985

(43) Date of publication of application: 03.06.87 Bulletin 87/23

(84) Designated Contracting States: AT BE CH DE ES FR GB GR IT LI LU NL SE

(71) Applicant: **ENIRICERCHE S.p.A.**
Corso Venezia 16
I-20121 Milan(IT)
0

(72) Inventor: **Pessi, Antonello**
Via Massaciuccoli, 19
Roma(IT)
Inventor: **Verdini, Antonio Silvio**
Via S. Martino, 12
I-00015-Monterotondo Roma(IT)
Inventor: **Viscomi, Giuseppe Claudio**
Via 8 Maggio, 23
I-00015-Monterotondo Roma(IT)

(74) Representative: **De Carli, Elda et al**
ENIRICERCHE S.p.A. BRELID Via F. Maritano, 26
I-20097 San Donato Milanese(IT)

## (54) Process for the solid-phase synthesis of retro-inverso peptides.

(57) A process for the solid-phase synthesis of a peptide containing at least one retro-inverso amide bond characterized in that :

a) the C-terminal partial peptide sequence up to the bond which has to be retro-inverted is synthesized by stepwise condensation of the amino acids suitably protected at the amino group as well as at the side chain functional groups, on a solid support which is insoluble in the reaction medium,

b) the dipeptide derivative of general formula (I) :

$$Fmoc-NH-\underset{R}{\underset{|}{CH}}-NH-CO-\underset{R_1}{\underset{|}{CH}}-COOH \qquad (I)$$

wherein
Fmoc is N-9-fluorenylmethoxycarbonyl, and
R and $R_1$, each independently, represent the side-chain residues of natural amino acids wherein the functional groups, if any, are suitably protected,
is coupled to the amino terminus of the partial peptide sequence covalently bound to the solid insoluble support,

c) the Fmoc amino-protecting group is cleaved from the peptide bound to the solid insoluble support,

EP 0 224 088 A2

d) the whole sequence of the desired retro-inverso peptide is then stepwise built up by conventional procedures

e) the thus obtained retro-inverso peptide is detached from the solid insoluble support and purified.

# PROCESS FOR THE SOLID-PHASE SYNTHESIS OF RETRO-INVERSO PEPTIDES

The present invention refers to a method for the solid-phase synthesis of peptides containing at least one retro-inverso amide bond.

In particular the present invention relates to a method for the solid-phase synthesis of retro-inverso peptides which is of particular value for the synthesis of retro-inverso peptides wherein the C-terminal partial peptide sequence up to the retro-inverso bond may contain aromatic amino acid residues and/or sulfur-containing amino acid residues.

Pharmacological or therapeutical use of natural peptides or their synthetic derivatives is often barred because of their poor in vivo stability.

The reasons for such limitation lay in the presence, in the peptide chain, of particular amide bonds which can easily and quickly be degraded by peptidase enzymes. According to known techniques, resistance to enzymatic degradation can be increased by reverting the direction of the labile amide bonds.

The reversal of the direction of a peptide bond, also named retro-inversion, i.e. from a partial structure

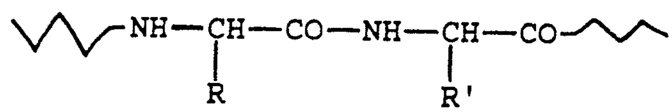

to a partial structure

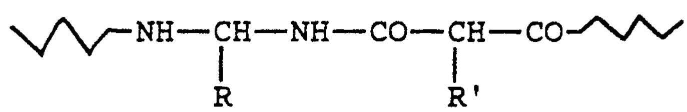

enhances the stability of the amide bond to enzymatic degradation and may lead to peptide analogues with an in vivo activity which lasts longer than the parent compound. The thus obtained analogues, named retro-inverso peptides, might therefore be suitably applied in the pharmaceutical and therapeutical fields.

The reversal of the direction of an amide bond in a peptide sequence, involves the modification of the two adjacent amino acid residues engaged in the retro-inversion and, in particular, the replacement of the amino acid residue nearer to the amino terminus of the reference peptide with a geminal diamino residue, and of the amino acid residue nearer to the carboxyl terminus with a malonyl or 2-substituted malonyl residue.

While the incorporation of a malonyl or 2-substituted malonyl residue in the peptide chain does not involve particular problems, the introduction of a gem-diamino residue requires the setting up of rather complicated synthetic procedures.

Italian patent application 22046 A/82 describes and claims a method for the solid-phase synthesis of peptides containing at least one retro-inverso amide bond which comprises the synthesis of the mono-acylated gem-diamino residue directly on the resin by the comlined use of a cross-linked polyamide resin and bis[(trifluoro-acetoxy)iodo]benzene (TIB).

This reactant,in fact, allows the direct conversion of primary amides of peptide chains synthetized on polymeric matrices and covalently bound to the solid supports into amines, thus overcoming the known problems.

However the process described in Italian patent application IT 22046 A/82 cannot satisfactorily be employed when retro-inverso peptides are desired wherein the C-terminal partial peptide sequence up to the bond which has to be retro-inverted, contains aromatic amino acid residues or sulfur-containing amino acid residues. The TIB reagent in fact, besides transforming the amides into amines, also brings about the oxidation of the aromatic amino acid residues such as tyrosine and tryptophan and of the sulfur-containing amino acid residues such as cysteine and methionine, possibly contained in the resin-bound partial peptide sequence. While the oxidation of the tyrosine residue may be avoided through suitable t-butyl protection of the phenol hydroxy group, the use of suitable protecting groups in the case of tryptophan or cysteine, does not prevent the side chain oxidation of the amino acid residues.

Methionine thioether group is transformed, in the reaction conditions described in Italian patent appication 22046 A/82, into the corresponding sulphoxide and can be restored by treatment of the retro-inverso peptide with N-methylmercaptoacetamide.

In the case of cysteine, furthermore, such treatments require reaction conditions which may affect peptide chain integrity.

An additional disadvantage of the above process concerns the retro-inversion of amide bonds which involve a proline residue.

As a matter of fact, the use of proline residues with D-configuration, owing to the cyclic nature of their side chain, leads to retro-inverso analogues which are topologically different from the reference peptides. It has now been found that it is possible to overcome the disadvantages of the known art by means of an easy and convenient process.

Object of the present invention is therefore a process for the solid-phase synthesis of peptides which contain at least one retro-inverso amide bond.

More particularly, object of the present invention is a process for the solid-phase synthesis of retro-inverso peptides wherein the C-terminal partial peptide sequence up to the bond which has to be retro-inverted, may contain aromatic amino acid residues and/or sulfur-containing amino acid residues.

According to the present invention, peptides containing at least one retro-inverso amide bond, wherein the C-terminal partial peptide sequence up to the bond to be retro-inverted, may contain aromatic amino acid residues and/or sulfur-containing amino acid residues, are synthesized through a solid-phase process characterized in that :

a) the C-terminal partial peptide sequence up to the bond which has to be retro-inverted, which may contain aromatic amino acid residues and/or sulfur-containing amino acid residues, is synthetized by stepwise condensation of the corresponding amino acids, suitably protected at the amino group as well as at the side chain functional groups, on a solid support which is insoluble in the reaction medium,

b) the dipeptide derivative of general formula (I) :

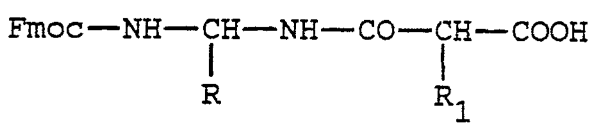

(I)

wherein

Fmoc is N-9-fluorenylmethoxycarbonyl, and

R and $R_1$, each independently, represent the side-chain residues of natural amino acids wherein the functional groups, if any, are suitably protected with known protecting groups,

is coupled to the amino terminus of the partial peptide sequence covalently bound to the solid insoluble support.

c) the Fmoc amino-protecting group is cleaved from the peptide bound to the solid insoluble support,

d) stepwise buildup of the entire peptide sequence is then completed by means of conventional procedures which provide, as the first step, coupling of the suitably selected amino acid and/or dipeptide derivative of formula (I) wherein the functional groups are suitably protected, to the geminal monoacylated diamino residue of the peptide chain attached to the solid insoluble support,

e) the thus obtained retro-inverso peptide is detached from the solid insoluble support by means of a suitable cleaving agent, and purified by conventional procedures.

Throughout the present specification, the abbreviations used for designating the amino acids and the protective groups are based on recommendations of the IUPAC-IUB Commission on Biochemical Nomenclature. For instance, Gly, Phe, Leu, Met, and Glp represents the "residues" of glycine, phenylalanine, leucine, methionine, and pyroglutamic acid, respectively. By the term "residue" is meant a moiety derived from the corresponding amino acid by eliminating the OH portion of the carboxyl group and the H portion of the amino group. The use of a "g" before the abbreviation means that in the corresponding amino acid residue the CO group has been replaced by a NH group (to give the corresponding gem-diamino residue), while the use of a "m" means that the NH group of the corresponding amino acid residue has been replaced by a CO group (to give the corresponding malonyl or 2-substituted malonyl residue).

According to the present invention, a stable polymer material appropriately functionalised is employed as the solid insoluble support for the stepwise assembly of the desired retro-inverso peptide.

More particularly polyamide or polystyrene resins can suitably be employed in our process.

Examples of suitable polyamide resins are, for instance, copoly[dimethylacrylamide-N-(t-butoxycarbonyl-$\beta$-alanyl)-N'-acryloylhexamethylenediamine] cross-linked with N,N'-bisacryloylethylenediamine,
copoly(dimethylacrylamide-acryloylsarcosine methyl ester) cross-linked with N,N'-bisacryloylethylenediamine and copoly(N-acryloylpyrrolidine-N'-acryloylhexamethylenediamine) cross-linked with N,N'-bisacryloylethylenediamine. Generally, a copoly(dimethylacrylamide-acryloylsarcosine methyl ester) resin cross-linked with N,N'-bisacryloylethylenediamine, with a residual sarcosine content of from about 0.1 and about 1.0 meq per gram is employed. Other polymeric materials with a different degree of cross-linking can be utilized conveniently, provided the synthetic polymer support used is freely permeated by the reactants and solvent media employed.

The resin is suitably functionalised by treatment with ethylenediamine according to known procedures - (see for instance R. Arshady et al. in J.Chem.Soc. Perkin I, (1981), pp.529-536). In particular the resin is shaken with excess ethylenediamine for 12-18 hours at room temperature. Then the excess ethylenediamine is removed and the resin is thoroughly washed with a polar inert solvent.

An internal standard, typically norleucine, may then be coupled to the $NH_2$ groups of the thus functionalised resin through reaction with a suitably protected symmetrical anhydride such as for instance $(Fmoc\text{-}Nle)_2O$, at room temperature for about 30-60 minutes, followed by removal of the protecting group. When, according to a preferred embodiment, N $^{\alpha}$-protection is achieved by using the N-9-fluorenylmethoxycarbonyl group, its removal is simply carried out by treatment with a piperidine/dimethylformamide 1/4 - (v/v) mixture at room temperature for few minutes.

A suitable linkage agent is then attached either to the amino-functionalised resin or to the amino group of the internal reference amino acid.

Linkage agents which are conveniently employed in the process of the present invention are those commonly used in conventional solid-phase peptide synthesis and preferably benzyl alcohol derivatives such as for instance 3-(4-hydroxymethylphenyl)propionic acid, (4-hydroxymethylphenoxy)acetic acid, [(4-hydroxymethyl-2-methoxy)phenoxy]acetic acid and 4-hydroxymethylbenzoic acid.

The introduction of the linkage agent is carried out using an activated form thereof. As an example, trichlorophenyl, 4-nitrophenyl, 1-benzotriazolyl, or pentafluorophenyl ester derivatives of 4-hydroxymethylbenzoic acid, (4-hydroxymethylphenoxy)acetic acid, [(4-hydroxymethyl-2-methoxy)phenoxy]acetic acid or 3-(4-hydroxymethylphenyl)propionic acid can suitably be used . Actually the reaction is carried out by contacting the functionalised resin with a dimethylformamide or tetrahydrofuran solution of the active ester of the linkage agent in the presence of equivalent proportion of a suitable condensation catalyst such as N-hydroxybenzotriazole or 3-hydroxy-4-oxo-3,4-dihydro-quinazoline, at room temperature for few hours.

In step a) of the above scheme, the peptide chain containing aromatic amino acid residues and/or sulfur-containing amino acid residues, is synthetized by stepwise condensing the amino acid residues suitably protected at the amino function as well as at the side functions with known protecting groups, to the thus functionalised resin.

The amino acid sequence is assembled one residue at a time attached to the insoluble polymeric support, and each cycle of amino-addition involves coupling and deprotection steps. Conventional protecting groups can be employed in the present process such as for instance the easily removable tert-butoxycarbonyl, tert-amyloxycarbonyl or N-9-fluorenylmethoxycarbonyl groups to block the N $^{\alpha}$-functions while more stable protecting groups which are not cleaved under the conditions used for intermediate $N^{\alpha}$-deprotection must be used for the side-chain functional group protection. Examples of such protecting groups which are however widely known in peptide chemistry are, for instance, benzyl or halo-, alkyl-or alkoxy-substituted benzyl groups for hydroxyl functions in tyrosine, threonine, glutamic acid ($\gamma$-carboxyl), aspartic acid ($\beta$-carboxyl) and serine, benzyloxycarbonyl or halo-, alkyl-or alkoxy-substituted benzyloxycarbonyl groups for $N^{\epsilon}$-protection in lysine, benzyloxycarbonyl, substituted benzyloxycarbonyl or tert-butoxycarbonyl groups for SH protection and the like protecting groups.

Preferably, however, the terminal amino group is protected with the N-9-fluorenylmethoxycarbonyl group and the side chain functions are protected with protecting groups which are stable under mild basic conditions but are labile in the reaction conditions used for cleaving the retro-inverso peptide from the polymer support, thus allowing cleavage from the resin with simultaneous removal of side chain and N-terminal protecting groups.

For the repeated couplings of step a) the thus protected amino acids are activated at the terminal carboxy group through formation of a symmetrical anhydride or an active ester according to techniques widely known in peptide chemistry. Preferably the activation is carried out through formation of the symmetrical anhydride of the terminal carboxy group.

Generally the esterification to the resin is carried out in the presence of a condensation catalyst as known in the art. According to a preferred embodiment,esterification to the resin is carried out in the presence of 4-dimethylaminopyridine which is known to be a particularly effective catalyst for ester bond formation under mild conditions. The reaction also requires the use of an equimolar amount of a nitrogen containing tertiary organic base such as for instance N-methyl-morpholine (NMM) or N-ethyl-morpholine - (NEM) which acts as the acid accepting agent. The reaction which is conveniently carried out at room temperature is generally complete in 1/2-2 hours.

The condensation of each amino acid is followed by washings with a polar inert organic solvent e.g. dimethylformamide, and by cleavage of the protecting group from the amino terminal residue. When, according to a preferred embodiment of the present invention, $N^{\alpha}$-protection is achieved by the use of Fmoc, its removal is easily carried out under mild basic conditions.

According to step b) of the above scheme a dipeptide derivative of general formula

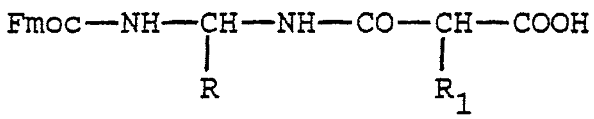

(I)

wherein Fmoc is the N-9-fluorenylmethoxycarbonyl group, and R and $R_1$, each independently, represent the side-chain of natural amino acids wherein the functional groups, if any, are suitably protected with known protecting groups, is then condensed to the amino terminus of the peptide segment covalently bonded to the resin which may contain aromatic amino acid residues and/or sulfur-containing amino acid residues.

The dipeptide of general formula (I) is activated at the terminal carboxyl group by the use of condensing agents such as, for instance, dicyclohexylcarbodiimide in the presence of N-hydroxybenzotriazole and then coupled to the resin-bound peptide by amide bond formation between the $NH_2$ group of the growing peptide chain and the activated acid group of the dipeptide of formula (I).

The amide bond formation may conveniently be carried out at room temperature (20-25°C). At the end of the reaction, the resin is washed with dimethylformamide, the excess solvent is removed and the Fmoc protecting group is then cleaved from the $NH_2$ residue of the resin bound peptide by treatment with a pyridine/dimethylformamide (1/4, v/v) mixture.

According to step d) of the present invention, the suitably protected amino acids and/or dipeptide derivatives of formuia (I) are then condensed according to the standard operation cycles reported above, till the desired peptide sequence is completed.

At the end of this reaction the retro-inverso peptide is detached from the resin by means of a suitable cleaving agent.

When the base-labile 4-hydroxymethylbenzoic acid is employed as the linkage agent, detachment of the retro-inverso peptide from the resin is carried out by treatment of the resin with saturated methanolic ammonia. The reaction which is carried out at a temperature of about 0 °C for 30 to 120 min. and at room temperature (20-25 °C) for 120 to 180 min., yields the desired retro-inverso peptide amide. When, on the other hand, an acid-labile linkage agent is employed, detachment of the end retro-inverso peptide from the resin by treatment with hydrofluoric or trifluoroacetic acid, affords the desired peptide in acidic form. The resin is then separated by filtration and washed with methanol to complete cleavage of the retro-inverso peptide. The filtrate and the mother liquors are combined and evaporated to dryness. The dry residue thus obtained is washed with an inert polar organic solvent and concentrated to dryness under vacuum at room temperature.

The retro-inverso peptide is then purified, if necessary, by conventional chromatographic techniques.

According to the present invention, the dipeptide derivative of general formula (I) is obtained by transforming the amino acids involved in the retro-inverso bond and then condensing the thus obtained amino acid derivatives in solution.

In particular the amino acid residue nearer to the amine terminus of the reference peptide which is $N^{\alpha}$ -protected with Fmoc, is transformed into a gem-diamino residue by using TIB for the direct conversion of the primary carboxyamidic group in amino group.

The amino acid residue nearer to the carboxyl terminus of the reference peptide is transformed into a malonic acid or 2-substituted malonic acid ester.

The thus transformed amino acid derivatives are then coupled in solution, in the presence of known condensing agents.

The condensation reaction is carried out at room temperature (20-25 °C) for a time sufficient to substantially complete the reaction. At the end of this time, the ester residue is removed from the thus obtained reaction product by a hydrogenolytic treatment which does not affect the stability of the protecting groups of the dipeptide derivative.

When benzyl esters are employed, for instance, a suspension of palladium on charcoal in a solution of ammonium formate in dimethylformamide is employed and the reaction is carried out at room temperature for a time ranging from 1 to 10 minutes.

The following working example further illustrates the process of the present invention without limiting its scope.

Example

Synthesis of pyroglutamylphenylalanyl-gem-diaminophenylalanylmalonylleucylmethionineamide : Glp-Phe-gPhe-mGly-Leu-Met-$NH_2$

The synthesis is carried out using a Beckman® (990 B type) Synthetizer and a copoly-(dimethylacrylamide-acryloylsarcosine methyl ester) resin crosslinked with N,N'-bisacryloylethylenediamine with a sarcosine content of 0.5 meq per gram.

The resin (0.5 g) is functionalised with ethylenediamine (16 ml) at room temperature for 16 hours. Then the condensation of the internal standard norleucine to the $NH_2$ groups of the functionalised resin is carried out. The reaction is performed by contacting a solution of $(Fmoc\text{-}Nle)_2O$ (0.9 mmol) in dimethylformamide - (DMF) (8 ml) with the resin and keeping the suspension at room temperature, under stirring, for 60 minutes.

The liquid phase is removed, the resin is washed with DMF and the linkage agent is then attached to the resin by the addition of a DMF solution of 4-hydroxymethylbenzoic acid 2,4,5-trichlorophenyl ester to the resin.

The reaction is carried out in the presence of N-hydroxybenzotriazole (HOBt) at room temperature for 60 minutes.

Then the liquid phase is removed, the resin is washed with DMF and the C-terminal methionine residue is condensed to the resin. The esterification reaction is carried out by charging $(Fmoc\text{-}Met)_2O$ (0.7 g, 0.9 mmol), N-methyl-morpholine (NMM) (0.091 g, 0.9 mmol), and 4-dimethylaminopyridine (DMAP) (0.011 g, 0.09 mmol) in the reactor.

The reaction is carried out at room temperature, under stirring, for 30 minutes.

At the end of this time, the Fmoc $N^{\alpha}$-protecting group is cleaved by treatment with piperidine/DMF 1/4 - (v/v).

The resin is washed with DMF and a solution of $(Fmoc\text{-}Leu)_2O$ (0.635 g, 0.9 mmol) in DMF (8 ml) is then added thereto. The condensation reaction is carried out, under stirring, for 60 minutes. Upon cleavage of the Fmoc group, the resin is washed with DMF and the excess solvent is removed.

Fmoc-gPhe-mGly-OH (0.400 g, 0.9 mmol) prepared as described below, is activated at the terminal carboxy group by reaction with DCC (0.184 g, 1 eq) and HOBt (0.122 g, 1 eq) dissolved in DMF (8 ml) and then condensed to the resin through acylation of the Leu -$NH_2$ group with the activated carboxyl group.

The acylation reaction is carried out under stirring at room temperature for 16 hours. The resin is washed with DMF (10 $\times$ 1 min.), the excess solvent is removed then the Fmoc protecting group is cleaved from the $NH_2$ residue. This reaction is carried out by treating the resin with a solution of piperidine/DMF 1/4 (v/v) (1 $\times$ 3 min. and 1 $\times$ 7 min.), at room temperature.

The resin is washed with DMF (10 $\times$ 1 min.) and the amino acid derivative $(Fmoc\text{-}Phe)_2O$ and the pyroglutamic acid pentachlorophenyl ester Glp-OPCP) are condenses thereto according to the working procedures described above. The thus synthesized hexapeptide is detached from the resin through ammonolysis.

The dry resin (0.5 g) is suspended in DMF (8 ml) and allowed to stand at room temperature for 30 minutes. Methanol (MeOH) (50 ml) is then added, the suspension is cooled to 0 °C and $NH_3$ is bubbled therein until the suspension is saturated.

The suspension is kept at 0 °C for two hours and at room temperature for further two hours.

Then the resin is removed by filtration, washed with methanol, the filtrate and the methanolic mother liquors are combined and evaporated to dryness.

The thus obtained residue is taken up in ethanol (50 ml), evaporated and concentrated to dryness under vacuum.

Cleavage of the hexapeptide from the resin affords a 98% yield.

The peptide dissolved in aqueous hexafluoroisopropanol is purified by reverse phase high pressure liquid chromatography with Lichroprep®RP-18 (25-40 µm Merck) as the stationary phase and eluting with $H_2O/CH_3CN$ (80/20, v/v).

The peptide, recovered by freeze-drying the peptide-containing fractions after removal of $CH_3CN$, shows the same properties of the reference peptide.

The hexapeptide has a m.p. of 261-65 °C.

$[\alpha]^{22}_{D}$ = + 10.0° (c=0.5 in DMF)

Elemental analysis (%) for $C_{36}H_{49}N_7O_7S$

Calculated C, 59.75; H, 6.77; N, 13.55

Found C, 59.77; H, 6.68; N, 13.47

Amino acid analysis

Calculated Glp, 1.00; Phe, 1.00; Leu, 1.00; Met, 1.00

Found Glp, 0.97; Phe, 0.98; Leu, 1.00; Met, 0.95

Tlc and hplc confirm that the product is homogeneous and $^1$H-nmr and FAB/MS analyses confirm the assigned structure.

Preparation of the starting material

a) Synthesis of N-9-fluorenylmethoxycarbonylphenylalanineamide : Fmoc-Phe-$NH_2$

Anhydrous tetrahydrofuran (THF)(70 ml) and Fmoc-Phe-OH (3.94g, 1.0 mol) are charged into a 250 ml-reaction flask equipped with stirring means.

To the obtained solution cooled to -15 °C and kept under nitrogen atmosphere, NMM (1.127g, 1.0 mol), isobutylchloroformate (1.58 ml, 1.0 mol), and, about two minutes later, aqueous ammonia (1.36 ml, 30% w/v, 1.0 mol) are added.

The thus obtained solution is kept at a temperature lower than -10 °C for one hour and then brought to room temperature (20-25°C).

The reaction mixture is then treated with an excess of 5% aqueous $NaHCO_3$. The precipitate which forms is recovered by filtration, washed with water (5 x 200 ml), dried over $P_2O_5$, washed with ethyl ether -($Et_2O$) (3 x 100 ml)and finally dried under vacuum.

The obtained compound has m.p. 174-76 °C, and $[\alpha]^{22}_{D}$ = + 20.9°(c=2.07, hexafluoroisopropanol).

Elemental analysis (%) for $C_{24}H_{22}N_2O_3$

Calculated C, 76.6; H, 5.7; N, 7.2

Found C, 76.3; H, 4.56; N, 7.1

The chromatographic analysis (tlc-h, hplc) does not show any impurities and $^1$H-nmr confirms the assigned structure.

b) Synthesis of N-9-fluorenylmethoxycarbonyl-gem-phenylalanine hydrochloride : Fmoc-gPhe-H•HCl

I,I-bis[(trifluoroacetoxy)iodo]benzene (TB) (4.73 g, 1.2 equiv.) in DMF (50 ml) is added to a solution of Fmoc-Phe-$NH_2$ (3.86 g, 1.0 eq) in anhydrous DMF (100 ml). The solution is kept at 5 °C until the reaction is complete. The disappearance of the starting amide is checked by hplc. Water (0.54 ml, 3.0 eq) is then added to the reaction mixture.

The reaction mixture is maintained at 20°C for about 4 hours, then the reaction is stopped by the addition of aqueous 4N HCl (1.0 eq).

The solvent is evaporated off to dryness, the obtained residue is dissolved in DMF (10 ml) and the reaction product is precipitated by the addition of excess anhydrous $Et_2O$.

The precipitate is recovered by filtration, washed with $Et_2O$ (5 x 50 ml) and then concentrated to dryness under vacuum.

A compound with $[\alpha]^{22}_{D}$ = -45.67°(C=2.06 in DMF) is obtained.
Elemental analysis (%) for $C_{23}H_{23}N_2O_2Cl$
Calculated C, 67.2; H, 5.6; N, 6.8
Found C, 66.9; H, 5.6; N, 6.7
Chromatographic analysis (tlc, hplc) does not show any impurities and $^1$H-nmr analysis confirms the assigned structure.

c) Synthesis of mono-tert-butyl-malonate: HO-mGly-OBu$^t$

1N NaOH (1.0 eq) is added to a solution of malonic acid methyl tert-butyl ester (20 g, 1.0 eq) in MeOH (50 ml). The thus obtained solution is kept at 5 °C until the hydrolysis is complete. Then, the solution is diluted with water (100 ml) and the organic solvent is evaporated off. The aqueous solution is washed with ethyl acetate (AcOEt) (2 × 200 ml) and brought to pH 2 by the addition of concentrated HCl. The obtained acid solution is extracted with AcOEt (3 × 150 ml).

The organic extracts are combined, concentrated to a small volume, dried over $MgSO_4$, filtered and evaporated to dryness.

The desired product is thus obtained as a colorless oil.
Elemental analysis (%) for $C_7H_{12}O_4$
Calculated C, 52.5; H, 7.5
Found C, 52.0; H, 7.3

d) Synthesis of malonic acid benzyl tert-butyl ester : BzlO-mGly-OBu$^t$

A solution of DMAP (2.56 g, 1.0 equiv.) in DMF (5 ml) and a solution of dicyclohexylcarbodiimide - (DCC) (4.76 g, 1.1 equiv.) in THF (10 ml) and benzyl alcohol (2.61 ml, 1.2 equiv.) are added to a solution of malonic acid mono-tert-butyl ester (3.5 g, 1.0 equiv.) in THF (10 ml). The reaction mixture is maintained at 20°C until the disappearance of the starting compound is confirmed by tlc analysis. The solvent is then evaporated off to dryness and the obtained residue is taken up in AcOEt (50 ml).

The obtained solution is washed sequentially with aqueous 5% $NaHCO_3$ (l00 ml), water (100 ml), aqueous 5% citric acid (100 ml) and finally water (100 ml).

The organic phase is concentrated under vacuum, dried over $MgSO_4$, filtered and evaporated to dryness. The desired product is obtained as a colorless oil.
Elemental analysis (%) for $C_{14}H_{18}O_4$
Calculated C, 67.2; H, 7.2
Found C, 67.0; H, 7.1
Mass spectrometry and $^1$H-nmr analysis confirm the assigned structure.

e) Synthesis of malonic acid mono-benzyl ester : HO-mGly-OBzl

Malonic acid benzyl tert-butyl ester (7 g, 1.0 equiv.) is dissolved in TFA/$CH_2Cl_2$ 50/50 (40 ml).

The mixture is kept at 20 °C for 30 min. The solvent is evaporated off to dryness and the thus obtained oily residue is triturated with n-hexane (100 ml).

The desired reaction product is obtained as a white hygroscopic crystalline solid melting at 46-48.5°C.
Elemental analysis (%) for $C_{10}H_{10}O_4$
Calculated C, 61.8; H, 5.2
Found C, 65.5; H, 5.0
Mass spectrometry and $^1$H-nmr analysis confirm the identity of the product.

f) Synthesis of N-9-fluorenylmethoxycarbonyl-gem-phenylalanylmalonic acid benzyl ester : Fmoc-gPhe-mGly-OBzl

Malonic acid mono-benzyl ester (1.96 g,1.0 equiv.) is dissolved in THF (15 ml). A solution of HOBt (1.66 g, 1.2 equiv.) in DMF (5 ml) and a solution of DCC (2.31 g, 1.1 equiv.) in THF (3 ml) are then added thereto.

The reaction is carried out at 0°C for 20 minutes and at room temperature (20-25 °C) for 40 minutes. Then the reaction mixture is filtered directly into a mixture of Fmoc-gPhe-H•HCl (2.0 g, 0.5 equiv.) and NMM (617 ml, 0.56 equiv.) in DMF (12 ml).

The thus obtained solution is maintained at room temperature until the condensation reaction is complete or substantially complete.

The solvent is then evaporated off from the reaction mixture and the solid residue is triturated with AcOEt (50 ml).

The solid residue is then sequentially washed with aqueous 5% $NaHCO_3$ (100 ml), water (50 ml), aqueous 5% citric acid (100 ml), and water (50 ml), dried over $P_2O_5$, washed with AcOEt (100 ml) and $Et_2O$ - (100 ml) and finally dried under vacuum.

A compound is thus obtained with m.p. 178-80°C and $[\alpha]^{22}_{D}$ = + 4.73° (c=1.06 in hexafluoroisopropanol).

Elemental analysis (%) for $C_{23}H_{30}O_5$

Calculated C, 66.6; H, 7.3; N, 6.8

Found C, 66.3; H, 7.2; N, 6.6

Chromatographic analyses (tlc, hplc) do not show any impurities and $^1$H-nmr analysis confirms the identity of the product.

g) <u>Synthesis of N-9-fluorenylmethoxycarbonylphenylalanylmalonic acid</u> : <u>Fmoc-gPhe-mGly-OH</u>

10% Palladium on charcoal (2g) is suspended in a solution of ammonium acetate (0.965 g, 4 equiv.) in DMF, Fmoc-gPhe-mGly-OBzl (2.0 g, 1.0 equiv.) is then added to the obtained suspension and the reaction mixture is kept at 20°C for 5 minutes.

The reaction mixture is then filtered, the solvent is evaporated off to dryness, and the residue is taken up in dioxane (100 ml) and freeze-dried.

A compound is obtained with m.p. 158-63 °C and $[\alpha]^{22}_{D}$ = +6.53°(c=2.02 in hexafluoroisopropanol).

Elemental analysis (%) for $C_{16}H_{24}N_2O_5$

Calculated C, 59.2; H, 7.4; N, 8.6

Found C, 59.0; H, 7.3; N, 8.7

Chromatographic analyses (tlc, hplc) do not reveal any impurities and $^1$H-nmr analysis confirms the identity of the product.

## Claims

1) A process for the solid-phase synthesis of a peptide containing at least one retro-inverso amide bond, wherein the C-terminal partial peptide sequence up to the bond to be retro-inverted, may contain aromatic amino acid residues and/or sulfur-containing amino acid residues, characterized in that :

a) the C-terminal partial peptide sequence up to the bond which has to be retro-inverted, which may contain aromatic amino acid residues and/or sulfur-containing amino acid residues, is synthesized by stepwise condensation of the amino acids suitably protected at the amino group as well as at the side chain functional groups, on a solid support which is insoluble in the reaction medium,

b) the dipeptide derivative of general formula (I) :

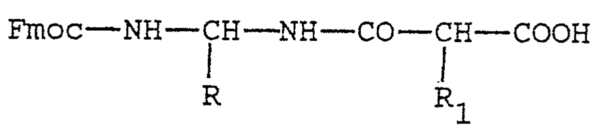

(I)

wherein

Fmoc is N-9-fluorenylmethoxycarbonyl, and

R and $R_1$, each independently, represent the side-chain groups, if any, are suitably protected with known protecting groups,

is coupled to the amino terminus of the partial peptide sequence covalently bound to the solid insoluble

support,

c) the Fmoc amino-protecting group is cleaved from the peptide bound to the solid insoluble support,

d) the whole sequence of the desired retro-inverso peptide is then stepwise built up by conventional procedures which involve, as the first step, coupling of the suitably selected amino acid and/or dipeptide derivative of formula (I) wherein the functional groups are suitably protected, to the geminal monoacylated diamino residue of the peptide chain attached to the solid insoluble support,

e) the thus obtained retro-inverso peptide is detached from the solid insoluble support by means of a suitable cleaving agent, and purified by conventional procedures.

2) A process according to claim 1 wherein the solid insoluble support is a polyamide or polystyrene resin.

3) A process according to claim 2, wherein the polyamide resin is copoly[dimethylacrylamide-N-(t-butoxycarbonyl-β-alanyl)-N'-acryloylhexamethylenediamine] cross-linked with N,N'-bisacryloylethylenediamine,
copoly(dimethylacrylamide-acryloylsarcosine methyl ester) cross-linked with N,N'-bisacryloylethylenediamine or copoly(N-acryloylpyrrolidine-N'-acryloylhexamethylenediamine) cross-linked with N,N'-bisacryloylethylenediamine.

4) A process according to claim 3, wherein the polyamide resin is copoly(dimethylacrylamide-acryloylsarcosine methyl ester) cross-linked with N,N'-bisacryloylethylenediamine.

5) A process according to claim 1, wherein, in step a), the esterification to the resin of the first amino acid is carried out in the presence of a condensation catalyst.

6) A process according to claim 5 wherein the catalyst is 4-dimethylaminopyridine.

7) A process according to claim 5, wherein the condensation reaction is carried out in the presence of an equimolar amount of a N-containing tertiary organic base.

8) A process according to claim 7 wherein the N-containing tertiary organic base is N-methyl-morpholine or N-ethyl-morpholine.

9) A process according to claim 1, wherein in step b) the condensation reaction is carried out through acylation of the amino terminal group of the resin bound peptide by the C-terminal carboxyl group of the dipeptide derivative of formula (I).

10) A process according to claim 9, wherein the C-terminal carboxyl group of the dipeptide derivative of formula (I) is activated through reaction with dicyclohexylcarbodiimide and N-hydroxybenzotriazole.

11) A process according to claim 1, wherein in step c), cleavage of the N-9-fluorenylmethoxycarbonyl -(Fmoc) protecting group is carried out by treatment with a mixture pyridine/DMF 1/4 (v/v).

12) A process according to claim 1, wherein in step e) detachment is carried out through ammonolysis using saturated methanolic ammonia, at about 0° for 2 hours and at room temperature for 2 hours.

13) A process according to claim 1, wherein in step e) purification is carried out by reverse-phase HPLC.

14) A peptide containing at least one retro-inverso amide bond whenever prepared by the method of any of preceding claims 1 to 13.